# EUROPEAN PATENT SPECIFICATION

(11) **EP 2 868 298 B1**
(45) Date of publication and mention of the grant of the patent: **11.01.2017**
(21) Application number: 13808747.3
(22) Date of filing: 27.06.2013
(51) Int. Cl.: A61F 5/56

(54) **ADHESIVE TAPE WITH NON-ADHESIVE PORTION AND USAGE METHOD THEREOF**
KLEBEBAND MIT NICHT HAFTENDEM TEIL UND VERWENDUNGSVERFAHREN DAFÜR
RUBAN ADHÉSIF À PARTIE NON ADHÉSIVE ET SON PROCÉDÉ D'UTILISATION

(30) Priority: 28.06.2012 US 201261665568 P
(43) Date of publication of application: 06.05.2015
(73) Proprietor: Somnics, Inc., Zhubei, Hsinchu 30261 (TW)
(72) Inventor: CHEN, Chung-Chu, Zhubei City Hsinchu County 30261 Taiwan (TW); HSIAO, Yung-Jing, Zhubei City Hsinchu County 30261 Taiwan (TW)
(74) Representative: Charrier Rapp & Liebau
(86) International application number: PCT/CN2013/078143
(87) International publication number: WO 2014/000660

(56) References cited:
- WO-A1-02/32350
- WO-A1-99/61089
- CN-A- 101 495 077
- DE-A1- 3 837 277
- FR-A1- 2 574 657
- JP-A- 2003 260 078
- JP-A- 2003 260 078
- US-A- 5 640 974
- US-A1- 2003 149 387
- US-A1- 2007 185 427
- US-A1- 2008 053 459

## Description

### FIELD OF THE INVENTION

The present invention is related to devices capable of reducing mouth breathing during sleep, and especially to an adhesive strip with non-adhesive band according to the preamble portion of claim 1 or 2. The present invention has been developed for use in patients with sleep-disordered breathing, for example, snoring and obstructive sleep apnea.

### BACKGROUND OF THE INVENTION

Snoring and other sleep-disordered breathing conditions affect millions of patients and their spouse, partners, and/or children. Snoring typically comes from open of mouth during sleeping. Mouth breathing also causes problems for Obstructive Sleep Apnea (OSA) treatment, for example, Constant Positive Airway Pressure (CPAP), generally because the air pressure could not maintain due to air leaking via the mouth.

There have been several attempts to solve this problem by using adhesive tape to cover the mouth. United States Patent Nos. 5,640,974 and 5,690,121 disclosed a chin support device in the form of a U or V-shaped piece of adhesive material having a base adhered to the user's chin below the bottom lip and two arms which extend up the face towards the cheeks to support the user's lower lip upwardly against the user's upper lip. However, the two arms are too remote from the center of the mouth and are ineffective to close the mouth without air leakage. The large area of the tape also makes it less comfortable and more noticeable.

A variation of the approach is disclosed in United States Patents 6,089,232. The patent discloses an anti snoring device in the form of an elastic fabric panel, having adhesive covering one surface and a narrow slit cut through the panel near the center of the panel. A further variation of the approach is disclosed in United States Patents 7,032,598 which disclosed a method comprising affixing a strip having an adhesive backing, extending from under the user's chin, over the center of the mouth. These devices could prevent the mouth from opening more effectively. However, due to the nature of their designs, the adhesive will contact over the upper and lower lips. Because the lip skin is thinner and more vulnerable, the adhesive may cause dry, cracked and peeling lips. It might even lead to bleeding if the adhesive pulls the dry skin off the lips. WO 99/61089 A1 discloses an apparatus for preventing or reducing the passage of air through a wearer's mouth. The apparatus includes a first portion adapted to be adhered above the wearer's upper lip, a second portion adapted to be adhered below the wearer's lower lip and at least one resilient joining member extending between the first and second portions. The joining member(s) are adapted to bias the first and second portions and thereby the upper and lower lips together to substantially seal the mouth.

WO 02/32350 A1 discloses a system to prevent snoring that can be used for people who snore and that comprises a slightly elastic strip whose width is smaller than the mouth. The central area on one of the sides of said strip has a lip protector and an adhesive is located on the two ends thereof. The outer part of the strip is placed in a central, vertical position relative to the mouth of the user. The strip adheres to the furrow between the lips and the mentolabial groove, the central area of the strip being located between the front edge of both lips.

JP 2003/260078 A provides an aromatic sticking member for preventing snore which makes comfortable sleeping, whereby the aromatic sticking member comprises a main body portion including an aromatic substance and sticking portions arranged so as to bridge across from an upper lip to a lower lip of a closed mouth provided on the above main body portion for sticking the above main body portion.

US 2007/185427 A1 discloses an adhesive bandage with reduced pain during removal, whose flat strip substrate has a sticking front, a non-sticking back and four sides. And, both areas of said sticking front and non-sticking back are greater than those of four sides; The feature is: a strip of non-glued area is disposed between both of opposite parallel lengthwise lateral sides and two strips of glued area are parallel juxtaposed on both lengthwise sides of said non-glued area respectively on said sticking front, wherein a pair of glue-less indentations are formed on both end sides of said non-glued area but not adjacent said glued area of said strip substrate and being flush with both crosswise end sides.

US 2003/149387 A1 discloses an anti-snoring devices, more specifically to externally worn anti-snoring devices covering essentially a wearer's mouth and being adhesively attached to a wearers skin in the peri-oral area are described.

None of the above devices allows mouth breathing, coughing or speaking during usage in a comfort way.

### SUMMARY OF THE INVENTION

Therefore, it is one object of the present invention to provide alternative and improved devices for reducing mouth breathing while substantially overcome one or more of the prior art deficiencies.

It is another object of the present invention to provide a small-sized device feel comfortable in usage and preventing the user's mouth from dry, peeling and/or bleeding.

Yet a further object of the present invention is to provide a device, which is flexible and will allow mouth breathing, coughing or speaking during usage.

The present invention provides an adhesive strip with non-adhesive band according to claim 1 or 2. The adhesive strip comprises a strip body and a non-adhesive band. The strip body has a first surface being adhesive and a second surface. The non-adhesive band is disposed on the first surface of the strip body to divide the strip body into an upper adhesive part and a lower adhesive part, wherein the non-adhesive band has an outer contour conforms to a contour of the strip body within their overlapping height and a height larger than a combined thickness of a user's lips in a mouth-closed condition.

In usage of the present adhesive strip with non-adhesive band, a horizontal center line of the non-adhesive band aligns with a horizontal center line between the user's lips, and the non-adhesive band covers a partial area of the user's mouth. The upper adhesive part and lower adhesive part are respectively attached unto skins above the user's upper lip and below the user's lower lip, and the upper adhesive part and lower adhesive part are not adhered to the user's lips. The non-adhesive band is not adhered to the lips' skin, and it allows interfacial movement (sliding) between the contact surface of the non-adhesive band and the lips' skin. Therefore, the device will not pull the lips' skin when the user try to move the lips and open the mouth and it will not cause dry, cracked or peeling and even bleeding.

The non-adhesive band has a width smaller than a width of the user's mouth, W, preferably the width of the non-adhesive band is (1/3) W.

In yet another embodiment, the present invention comprises of an adhesive strip with non-adhesive band whereas the non-adhesive band is flexible and allows the lips to move more freely when the user intends to open the mouth to breathe, cough or speak.

In still another embodiment, the present invention comprises of an adhesive strip with non-adhesive band whereas the non-adhesive band has a low-friction surface that allows the lips to move more freely when the user intends to open the mouth to breathe, cough or speak.

In yet another embodiment, the present invention comprises of an adhesive strip with non-adhesive band whereas the non-adhesive band has a hydrophobic surface that repels liquid (like saliva) and reduces the chance that liquid enters the interface between the adhesive strip and reduces the adhesiveness.

The non-adhesive band only blocks part of the opening of the mouth, and is configured to offset from a central portion of the mouth. This may allow the user to drink more easily even when the present adhesive strip is attached to the mouth.

### BRIEF DESCRIPTION OF THE DRAWINGS

Fig. 1A through Fig. 1E shows a first embodiment of the present adhesive strip with non-adhesive band.
Fig. 2A1 through Fig. 2D shows a first variation of the first embodiment of the present invention.
Fig. 3A through Fig. 3E shows application diagrams and a method of applying an adhesive strips with non-adhesive band according to the first variation of the first embodiment of the present invention.
Fig. 4A through Fig. 4D respectively shows front views of second through fifth variations of the first embodiment of the present invention.
Fig. 5A through Fig. 5C respectively shows cross-section views of sixth through eighth variations of the first embodiment of the present invention.
Fig. 6A shows a cross-section view of a second embodiment of the present invention in usage.
Fig. 6B through Fig. 6D respectively shows cross-section views of first through third variations of the second embodiment of the present invention.
Fig. 7A shows a front view of a third embodiment of an adhesive strip with non-adhesive band in usage.
Fig. 7B through Fig. 7E respectively shows front views of first through fourth variations of the third embodiment shown in Fig. 7A, the variation shown in Fig. 7B being part of the invention.
Fig. 8A shows a front view of a fourth embodiment of the present invention in usage.
Fig. 8B through Fig. 8E respectively shows front views of first through fifth variations of the fourth embodiment of the present invention.
Fig. 9A shows a front view of a fifth embodiment of the present invention in usage.
Fig. 9B through Fig. 9D respectively shows front views of first through fifth variations of the fifth embodiment of the present invention.

### DESCRIPTION OF REFERENCE NUMBERALS

10a----upper lip
10b----lower lip
12----flow passage
14----chin
100, 500, 502, 504, 600, 601, 602, 603
----adhesive strip with non-adhesive band
120, 200, 401, 402, 403, 404, 520 ---- adhesive strip
100a, 200a, 520a, 620, 621, 622, 623 ---- upper adhesive part
100b, 200b, 520b, 630, 631, 632, 633 ---- lower adhesive part
510'----band adhesive layer
522a, 524a, 620----upper strip adhesive layer
522b, 524b, 630----lower strip adhesive layer
110, 210, 410, 420, 430, 440, 510, 512, 610, 611, 612, 613, 710, 711,
712, 713, 714, 810, 811, 812, 813, 814, 815, 910, 911, 912, 913
----non-adhesive band
110a----obstructive mouth opening area
110b----non-obstructive mouth opening area
140 500a, 504a----base
170----concave edge
180----convex edge
190----protruding tab
230----releasing tab
232----applying tab
234----notch
236----horizontal center line
238----vertical center line
502a, 600a, 601a, 602a, 603a----partial adhesive strip
514, 610, 611, 612, 613----non-adhesive band
611a----low-friction coefficient layer
612a----hydrophobic layer
623a, 633a----thinner edges
700, 701, 702, 703, 704, 800, 801, 802, 803, 804, 805, 900, 901, 902, 903----adhesive strip
710, 711, 712, 713, 714, 810, 811, 812, 813, 814, 815, 910, 911, 912, 913----non-adhesive band
720, 721, 722, 723, 724, 820, 821, 822, 823, 824, 825, 920, 921, 922
923----parafiltrum adhesive part
730, 731, 732, 733, 734, 830, 831, 832, 833, 834, 835, 930, 931, 932, 933----chin adhesive part
740, 741, 742, 743, 744, 840, 841, 842, 843, 844, 845, 941, 942---- concave edge
760, 770, 761, 762, 763, 764, 771, 772, 774----edge
860, 861, 862, 865----convex bottom edge
863, 864----concave bottom edge
940----convex edge
943----flat edge
750, 751, 752, 753, 754, 783, 784, 784, 850, 851, 852, 870, 871, 872, 853, 854, 855, 873, 874, 875, 950, 951, 952, 953, 960, 961, 962, 963 ----cut
794, 855, 970, 973----opening
930a, 931a, 932a----left extension
930b, 931b, 932b----right extension
930c, 931c, 932c, 933c----bottom extension
4011, 4021, 4031--left edge
4012, 4022, 4032----right edge
4101, 4201, 4202----protruding tab
4043----non-adhesive tab

### DETAILED DESCRIPTION OF THE INVENTION

Reference will now be made in detail to the present exemplary embodiments, examples of which are illustrated in the accompanying drawings.

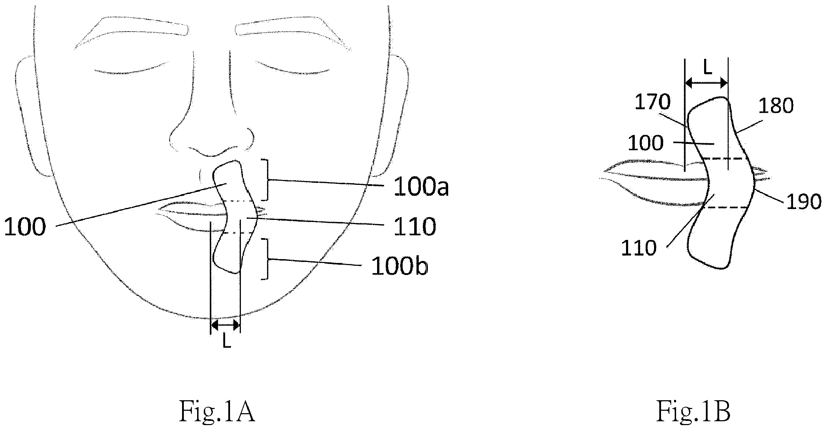

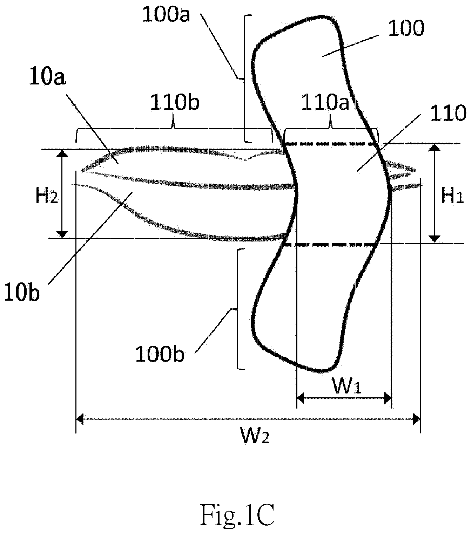

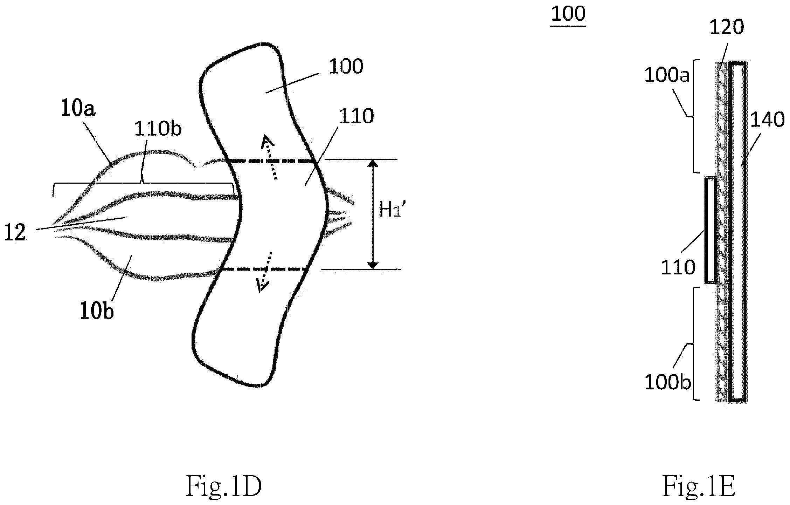

Fig. 1A through Fig. 1E shows a first embodiment of the present adhesive strip with non-adhesive band, in which Fig. 1A through Fig. 1D shows front views of an adhesive strip with non-adhesive band 100 according to the first embodiment, Fig. 1E shows a cross-section view of the adhesive strip with non-adhesive band. Firstly, refer to Fig. 1B and Fig. 1E, in the first embodiment, the adhesive strip with non-adhesive band 100 comprises an adhesive strip 120 and a non-adhesive band 110 across a central area of a first surface of the adhesive strip 120. The non-adhesive band 110 divides the adhesive strip 120 into an upper adhesive part 100a and a lower adhesive part 100b. The non-adhesive band 110 has an outer contour conforming to the contour of the adhesive strip 100 within their overlapping height. A base 140 is disposed on a second surface of the adhesive strip 120 opposing to the non-adhesive band 110. The base 140 has a configuration conforming to the shape of the adhesive strip 120. The non-adhesive band 110 has a concave edge 170 centered with a recess and a convex edge 180 centered with a convex portion. The concave edge 170 is opposite to the convex edge 180, and the convex edge 180 has a protruding tab 190 on the non-adhesive band 110. When the non-adhesive band 100 is applied to the user's mouth, the non-adhesive band 110 covers the user's lips, the upper adhesive part 100a and the lower adhesive part 100b are temporarily attached onto skins above the upper lip 10a and below the lower lip 10b, respectively. The upper adhesive part 100a and lower adhesive part 100b are not adhered to the lips. It is preferable that the concave edge 170 of the adhesive strip with non-adhesive band 100 faces a central portion of the user's mouth while the convex edge 180 faces one of corners of the user's mouth. A vertical center line of the adhesive strip with non-adhesive band 100 preferably has an offset L from a vertical center line of the lips (see Fig. 1B). The offset L is preferably (1/3) width of the user's mouth, W₂ (see Fig. 1C). The offset L could prevent the adhesive strip with non-adhesive band 100 from blocking the central portion of the mouth. Nevertheless, the use of the present adhesive strip with non-adhesive band 100 is not limited thereto. The offset L could be 0≦L≦W₂. The protruding tab 190 of the non-adhesive band 110 can be served as a handler, and the adhesive strip with non-adhesive band 100 after usage can be torn off the user's mouth by lifting the protruding tab 190. The base 140 provides supporting strength to maintain the shape of the adhesive strip 120.

Please refer to Fig. 1C and Fig. 1D, the non-adhesive band 110 has a width, W₁, which is smaller than the width of a user's lips, W₂, and W₁ is preferably about or smaller than 1/3 of W₂. This configuration provides an obstructive mouth opening area 110a covered by the non-adhesive band 110 besides a non-obstructive mouth opening area 110b. The non-obstructive mouth opening area 110b preserves an unblocked flow passage 12 between the upper and lower lips when the user intends to open the mouth to breathe, cough or speak. The non-adhesive band 110 has a height H₁ which is about or higher than the combined height H₂ of the upper and lower lips in mouth-closed condition so that the upper adhesive part 100a and lower adhesive part 100b will not be adhered to the user's lips. Because the non-adhesive band 110 will not adhere to the lips' skin, it allows interfacial movement (sliding) between the contact surfaces of the non-adhesive band 110 and the lips' skin. Therefore, the present adhesive strip with non-adhesive band 100 will not pull the lips' skin when the user tries to move the lips and open the mouth, and it will not cause dry, cracked or peeling, even bleeding of lips. The non-adhesive band 110 can be made of elastic materials with various thickness and flexibility. Potential materials of the non-adhesive band 110 include polyurethane (PU), polyethylene (PE), silicone, cloth, non-woven cloth, polyethylene terephthalate (PET), paper, as well as other flexible thin films. When the user intends to open the mouth by exerting force on muscles around the lips or on the lateral pterygoid muscles to depress the mandible (lower jaw), the elastic non-adhesive band 110 is extended to a height H₁' larger than its original height H₁. The mouth is opened and creates a flow passage 12 within the non-obstructive mouth opening area 110b allows the user to breathe, cough or speak more freely. When the user relaxes (without exerting force on muscles), the elastic non-adhesive band 110 will return to its original height H₁ and keep the mouth in closed condition again, as shown in Fig. 1C.

In an optional implementation, the surface of the non-adhesive band 110 contacting with the user's lips may be a low-friction surface with a friction coefficient less than 0.5 to allow the lips to move more freely when the user intends to open the mouth to breathe, cough or speak. Moisture may cause reduction of adhesiveness of the adhesive strip 120 and, in turn, cause the adhesive strip 120 losing its capability to close the mouth without air leakage. The surface of the non-adhesive band 110 contacting the user's lips may be a hydrophobic surface that repels liquid (like saliva) and prevents it from entering the interface between the adhesive strip 120 and the skin, and preventing the reduction of adhesiveness of the adhesive strip 120.

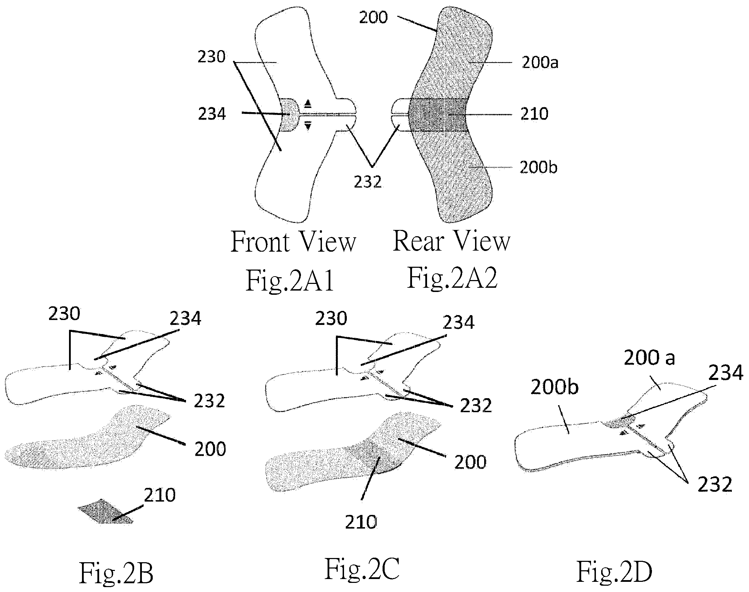

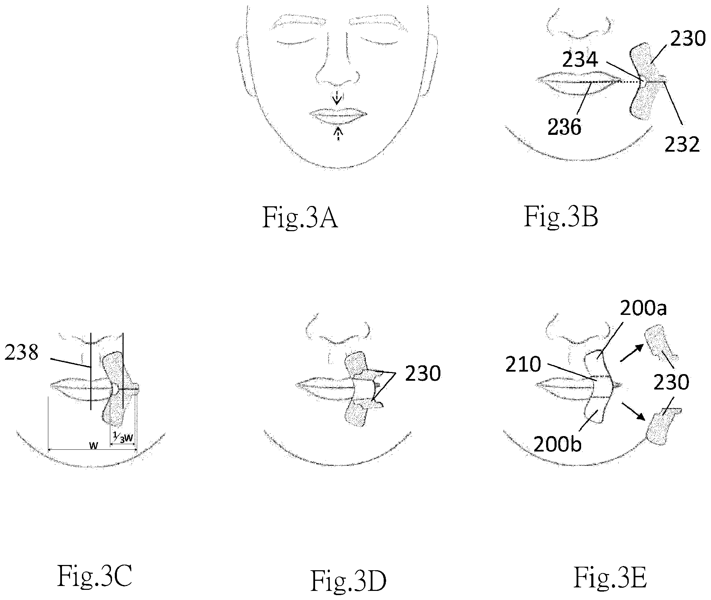

Fig. 2A1 through Fig. 2D shows front view, rear view, exploded view, partial exploded view and an assembly of a first variation of the first embodiment of the present invention, respectively. The present invention can use a releasing support instead of the base 140 of the adhesive strip with non-adhesive band 100. According to the first variation, the present invention provides an adhesive strip with non-adhesive band including an adhesive strip 200 and a non-adhesive band 210 across a central area of a first surface of the adhesive strip 200 and a releasing tab 230 disposed on a second surface of the adhesive strip 200 opposing to the non-adhesive band 210. The non-adhesive band 210 has an outer contour conforming to the contour of adhesive strip 200, and the non-adhesive band 210 divides the adhesive strip 200 into an upper adhesive part 200a and a lower adhesive part 200b. The adhesive strip 200 has a concave edge centered with a recess and a convex edge centered with a convex portion. As shown in Fig. 2A1, the releasing tab 230 is preferably composed of a pair of sub-releasing tabs each of them symmetrically attached unto one half area of the second surface of the adhesive strip 200 such that the releasing tab 230 forms an applying tab 232 protruding from an edge of the central area of the adhesive strip 200 and a notch 234 formed on the opposite edge of the central area of the adhesive strip 200. Nevertheless, the structure of the present adhesive strip with non-adhesive band is not limited thereto. The releasing tab 230 could be a single piece integrally formed. When the adhesive strip with non-adhesive band of the first variation is applied to a user's mouth, the applying tab 232 and notch 234 can be served as marks to align the horizontal center line of the non-adhesive band 210 with the horizontal center line between the upper and lower lips, and the non-adhesive band 210 covers the user's lips. The upper adhesive part 200a and the lower adhesive part 200b are temporarily adhered unto skins above the user's upper lip and below the user's lower lip, respectively. The upper adhesive part 200a and lower adhesive part 200b are not adhered to the use's lips. It is preferable that the concave edge of the adhesive strip with non-adhesive band faces the central area of the mouth and the convex edge of the adhesive strip with non-adhesive band faces one of corners of the mouth edge. A vertical center line of the adhesive strip with non-adhesive band preferably has an offset L from a vertical center line of the lips. The offset L is preferably (1/3) width of the user's mouth, W₂ (see Fig. 3C). The offset L could prevent the adhesive strip with non-adhesive band from blocking the central portion of the mouth. Nevertheless, the use of the present adhesive strip with non-adhesive band is not limited thereto. The offset L could be 0≦L≦(1/2)W. The releasing tab 230 can be torn off from the second surface of the adhesive strip 200 by lifting the applying tab 232 after the adhesive strip with non-adhesive band is applied to the user's mouth.

Except for the releasing tab 230 instead of the base 140, the rest elements of the first variation are the same with those of the first embodiment.

A method for preventing mouth from involuntary opening will be exemplary illustrated with the first variation of the first embodiment. Fig. 3A through Fig. 3E shows application diagrams and a method of applying the adhesive strips with non-adhesive band according to the first variation of the first embodiment to prevent the mouth from involuntary opening. The method comprises closing the lips lightly by a user (referring to Fig. 3A), next please refer to Fig. 3B, holding the adhesive strip with non-adhesive band besides one of corners of the user's mouth and the first surface of the adhesive strip 200 is faced with the user, the concave edge of the adhesive strip with non-adhesive band is faced with the central portion of the user's mouth, aligning the applying tab 232 and notch 234 with the horizontal center line of the user's lips. Please refer to Fig. 3C, horizontally moving the adhesive strip with non-adhesive band close to the corner of user's mouth and aligning the adhesive strips with non-adhesive band with a vertical center line of the user's lips with an offset L, attaching the upper adhesive part 200a to the skin above the upper lip and attaching the lower adhesive part 200b to the skin below the lower lip. The offset L is preferably 1/3 width of the user's lips, W, such that the adhesive strip with non-adhesive band does not block the central portion of the mouth. Continually, referring to Fig. 3D, lifting the applying tabs 232 in a direction of the vertical center line of the adhesive strip with non-adhesive band. Finally, referring to Fig. 3E, releasing the releasing support 230 from the second surface of the adhesive strip 200 with lifting the applying tabs 232.

Please note that the present method for preventing mouth from involuntary opening is not limited to the method illustrated with the first variation of the first embodiment, for example, the non-adhesive band can cover other portion of the user's mouth so that the offset L between the vertical center line of the non-adhesive band and the vertical center line of the mouth is 0≦L≦ (1/2) W, wherein W is a width of the user's mouth.

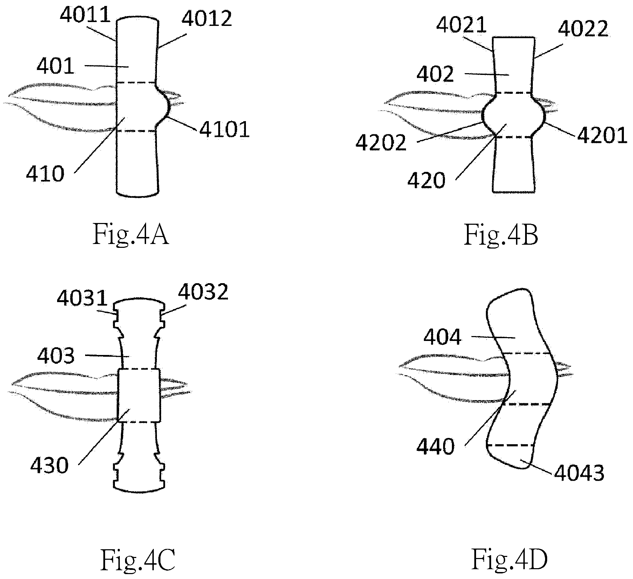

Please refer to Figs. 4A to 4D. Figs. 4A to 4D demonstrate front views of application diagrams of various adhesive strips with non-adhesive bands according to second variation to fifth variation of the first embodiment. Fig. 4A shows an adhesive strip with non-adhesive band which comprises of an adhesive strip 401 and a non-adhesive band 410 across a central portion of the adhesive strip 401. The adhesive strip 401 may have unsymmetrical left edge 4011 and right edge 4012. The non-adhesive band 410 may have a protruding tab 4101 on one of the unsymmetrical left and edges 4011 and 4012. A method for using the adhesive strip with non-adhesive band of the second variation is the same with that of the adhesive strip with non-adhesive band 100 of the first embodiment. For example, in usage, the protruding tab 4101 may be close to one of corners of the user's mouth so that the adhesive strip with non-adhesive band is favorably torn off the user's mouth with lifting the protruding tab 4101 after the usage. Fig. 4B shows an adhesive strip with non-adhesive band which comprises of an adhesive strip 402 and a non-adhesive band 420 across a central portion of the adhesive strip 402. The adhesive strip 402 may have symmetrical left edge 4021 and right edge 4022. The non-adhesive band 420 may have two protruding tabs, 4201 and 4202, on both of the symmetrical left and right edges 4021 and 4022, either of which can be used as a handler to lift the adhesive strip 402 after usage. Fig. 4C shows an adhesive strip with non-adhesive band which comprises of an adhesive strip 403 and a non-adhesive band 430 across a central portion of the adhesive strip 403. The adhesive strip 403 may have left and right zigzag edges 4031 and 4032. The left and right zigzag edges 4031 and 4032 increase contacting interface between the skin and the adhesive strip 403. The zigzag design may spread out the force applied on the adhesive strip 403 when a user tries to move the lips, and thus it may reduce the stress applied over the skin nearby and can be more comfortable. A method for using the adhesive strip with non-adhesive band of the fourth variation is the same with that of the adhesive strip with non-adhesive band 100 of the first embodiment. Fig. 4D shows an adhesive strip with non-adhesive band which comprises of an adhesive strip 404 and a non-adhesive band 440 across a central portion of the adhesive strip 404. The adhesive strip 404 may further have a non-adhesive tab 4043 on top or/and bottom edges of the adhesive strip 404, which can be used as a handler to lift the adhesive strip 404 after usage. Except for the non-adhesive tab 4043, the adhesive strip 404 may have a configuration substantially the same with that of the adhesive strip 100 of the first embodiment. A method for using the adhesive strip with non-adhesive band of the fifth variation is the same with that of the adhesive strip with non-adhesive band 100 of the first embodiment.

The like elements of the second to fifth variations are substantially the same with those of the first embodiment in size and materials. Similarly, in usage of these variations, the vertical center line of the adhesive strip with non-adhesive band preferably has an offset L from the vertical center line of the user's mouth. The offset L could be 0≦L≦(1/2)W, wherein W is a width of the user's mouth. Besides, a base or releasing support may be optionally disposed on a surface of the adhesive strips opposing to the non-adhesive bands as the first embodiment or the first variation does.

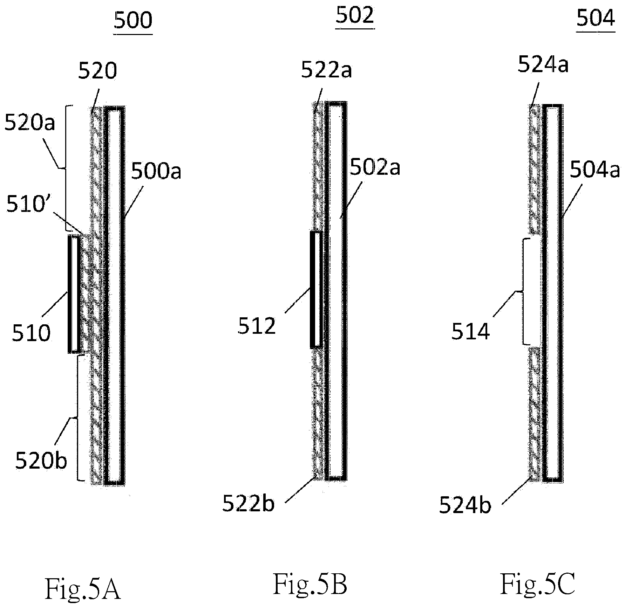

Figs. 5A to 5C demonstrate cross-section views of sixth to eighth variations of the first embodiment of the present invention. Fig. 5A demonstrates a cross-section view of an adhesive strip with non-adhesive band 500 of the sixth variation. In the sixth variation, the adhesive strip with non-adhesive band 500 comprises an adhesive strip 520 and a non-adhesive band 510 covers a central portion of a first surface of the adhesive strip 520 to divide the adhesive strip 520 into an upper adhesive part 520a and a lower adhesive part 520b. A band adhesive layer 510' with an outer contour conforming to the contour of the non-adhesive band 510 is placed between the adhesive strip 520 and the non-adhesive band 510. A base 500a is placed on a second surface of the adhesive strip 520 opposing to the non-adhesive band 510. The like elements of the sixth variation are substantially the same with those of the second to fifth variations and the first embodiment in size and materials. Their usages are also the same. Besides, a releasing support as the releasing tab 230 shown in Figs. 2A1 to 2D can be used instead of the base 500a. Fig. 5B demonstrates a cross-section view of an adhesive strip with non-adhesive band 502 of the seventh variation. In the seventh variation, a partial adhesive strip 502a, which has an upper strip adhesive layer 522a and a lower strip adhesive layer 522b formed on its one surface, is provided. A non-adhesive band 512 is bonded non-adhesively (such as ultrasonic bonding, thermal bonding, electrostatic bonding, etc.) to a central part of the partial adhesive strip 502a without any adhesive layer. The upper strip adhesive layer 522a, the lower strip adhesive layer 522b and the non-adhesive band 512 are formed on the same surface of the partial adhesive strip 502a. Thus the non-adhesive band 512 divides the partial adhesive strip 502a into an upper adhesive part accommodating the upper strip adhesive layer 522a and a lower adhesive part accommodating the lower strip adhesive layer 522b. The like elements of the seventh variation are substantially the same with those of the second to fifth variations and the first embodiment in size and materials. Their usages are also the same. Fig. 5C demonstrates a cross-section view of an adhesive strip with non-adhesive band 504 of the eighth variation. In the eighth variation, a partial adhesive strip 504a, which has an upper strip adhesive layer 524a and a lower strip adhesive layer 524b, is provided. A central part of the partial adhesive strip 504a without any adhesive layer forms a non-adhesive band 514. Thus the non-adhesive band 514 divides the partial adhesive strip 504a into an upper adhesive part accommodating upper strip adhesive layer 524a and a lower adhesive part accommodating lower strip adhesive layer 524b. The like elements of the eighth variation are substantially the same with those of the second to fifth variations and the first embodiment in size and materials. Their usages are also the same.

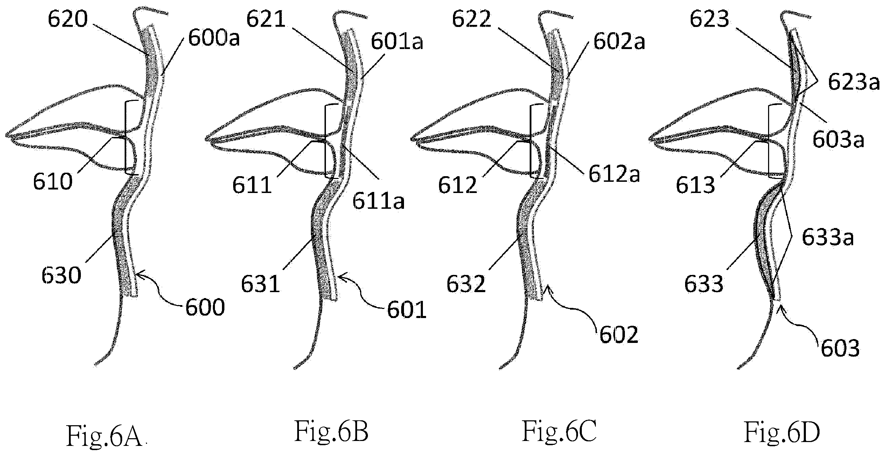

Fig. 6A demonstrates a cross-section view of an application diagram of an adhesive strip with non-adhesive band 600 according to a second embodiment of the present invention. In the second embodiment, a partial adhesive strip 600a, which has an upper strip adhesive layer 620 and a lower strip adhesive layer 630, is provided. The lower strip adhesive layer 630 may be longer than the upper strip adhesive layer 620 to accommodate various dimensions of different user's chins. A central part of the partial adhesive strip 600 without any adhesive layer forms a non-adhesive band 610. The non-adhesive band 610 covers the upper and lower lips and the lips are untouched by the upper and lower strip adhesive layers 620 and 630 so that the upper and lower strip adhesive layers 620 and 630 will not cause drying, cracking or peeling skin of the lips. Potential materials of the partial adhesive strip 600a include polyurethane (PU), polyethylene (PE), silicone, cloth, non-woven cloth, polyethylene terephthalate (PET), paper, as well as other flexible thin films. Except for the lower strip adhesive layer 630 longer than the upper strip adhesive layer 620, the rest elements of the second embodiment are substantially the same with those of the first embodiment and its variations in shape, size and materials. Their methods are also the same.

Figs. 6B to 6D are cross-section views of application diagrams of first to third variations of the second embodiment. Fig. 6B shows a cross-section view of an application diagram of an adhesive strip with non-adhesive band 601 of the first variation. In the first variation as shown in Fig. 6B, a partial adhesive strip 601 having an upper strip adhesive layer 621 and a lower strip adhesive layer 631 is provided. A central part of the partial adhesive strip 601 without any adhesive layer forms a non-adhesive band 611. The non-adhesive band 611 may further comprise a low-friction layer 611a with a friction coefficient less than 0.5 that allows the lips to move more freely when the user intends to open the mouth to breathe, cough or speak. The lower strip adhesive layer 631 may be longer than the upper strip adhesive layer 621 to accommodate various dimensions of different user's chins. The like elements of the adhesive strip with non-adhesive band 601 are substantially the same with those of the adhesive strip with non-adhesive band 600 in shape, size and materials. Their methods are also the same. Fig. 6C demonstrates a cross-section view of an application diagram of an adhesive strip with non-adhesive band 602 of the second variation. In the second variation, a partial adhesive strip 602a having an upper strip adhesive layer 622 and a lower strip adhesive layer 632 is provided. A central part of the partial adhesive strip 602a without any adhesive layer forms a non-adhesive band 612. Moisture may cause reduction of adhesiveness of the adhesive layers and, in turn, cause the adhesive strip losing its capability to close the mouth without air leakage. The non-adhesive band 612 may further comprise a hydrophobic layer 612a that repels liquid (like saliva) and prevents it from entering the interface between the adhesive strip and the skin. The like elements of the adhesive strip with non-adhesive band 602 are substantially the same with those of the adhesive strip with non-adhesive band 600 in shape, size and materials. Their methods are also the same. In an optional implementation, the non-adhesive band 612 may further comprise a low-friction and hydrophobic surface with a friction coefficient less than 0.5 (not shown in the drawing) to allows the lips to move more freely when the user intends to open the mouth to breathe, cough or speak, and preventing the reduction of adhesiveness of the upper strip adhesive layer 622 and the lower strip adhesive layer 632. Fig. 6D demonstrates a cross-section view of an application diagram of an adhesive strip with non-adhesive band 603 of the third variation. In the third variation, a partial adhesive strip 603 having an upper strip adhesive layer 623 and a lower strip adhesive layer 633 is provided. A central part 603a of the partial adhesive strip 603 without any adhesive layer forms a non-adhesive band 613. Moisture may cause reduction of adhesiveness of the adhesive layers and, in turn, cause the adhesive strip losing its capability to close the mouth without air leakage. The upper and lower strip adhesive layers 623 and 633 may further comprise thinner edges 623a and 633a than their other portions so that the smaller thickness will reduce the contacting areas of the upper and lower strip adhesive layers 623 and 633 to absorb moisture, and liquid (like saliva) may have less chance to enter the interface between the upper and lower strip adhesive layers 623 and 633 and the skin. The like elements of the adhesive strip with non-adhesive band 603 are substantially the same with those of the adhesive strip with non-adhesive band 600 in shape, size and materials. Their methods are also the same.

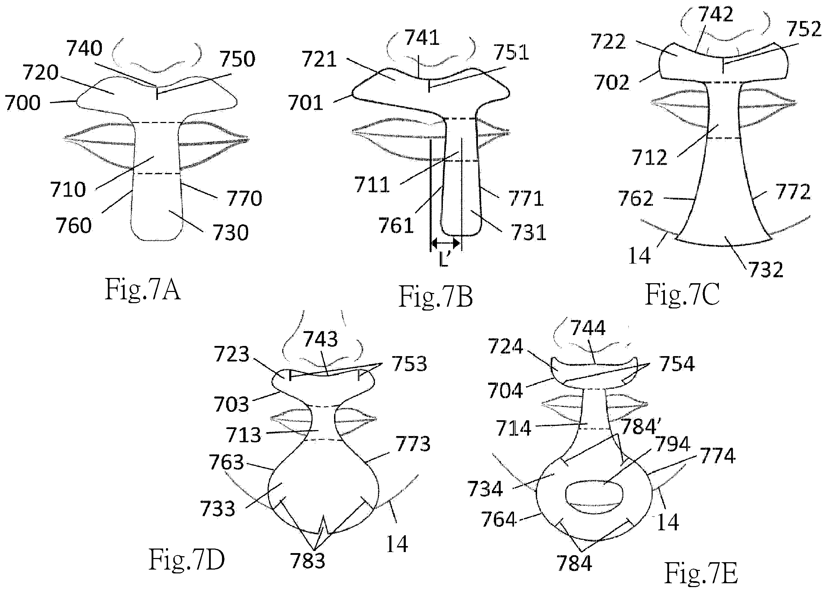

Fig. 7A demonstrates a front view of an application diagram of an adhesive strip with non-adhesive band 700 according to a third embodiment. The adhesive strip with non-adhesive band 700 comprises of a parafiltrum adhesive part 720, a chin adhesive part 730, and a non-adhesive band 710 across a mid portion of a first surface of the adhesive strip with non-adhesive band 700. The parafiltrum adhesive part 720 may have a width comparable to the width of the lips to increase adhesion. The parafiltrum adhesive part 720 may further have a concave edge 740 to accommodate the shape of the nose. The parafiltrum adhesive part 720 may also have one or more cuts 750 which may improve its conformity to various contours of different user's parafiltrum. The non-adhesive band 710 is smaller than the width of the lips, and preferably about or smaller than 1/3 of the width of the lips. The chin adhesive part 730 may extend from the non-adhesive band 710 downwardly below the lips. The chin adhesive part 730 may have a width smaller than the width of the lips. The adhesive strip 700 may further have symmetrical edges 760 and 770. The non-adhesive band 710 can be made of elastic materials with various thickness and flexibility. Potential materials of the non-adhesive band 710 include polyurethane (PU), polyethylene (PE), silicone, cloth, non-woven cloth, polyethylene terephthalate (PET), paper, as well as other flexible thin films. In an optional implementation, the surface of the non-adhesive band 710 contacting with the user's lips may be a low-friction surface with a friction coefficient less than 0.5 to allow the lips to move more freely when the user intends to open the mouth to breathe, cough or speak. Moisture may cause reduction of adhesiveness of the adhesive strip 700 and, in turn, cause the adhesive strip 700 losing its capability to close the mouth without air leakage. The surface of the non-adhesive band 710 contacting the user's lips may be a hydrophobic surface that repels liquid (like saliva) and prevents it from entering the interface between the adhesive strip 700 and the skin, and preventing the reduction of adhesiveness of the adhesive strip 700.

In usage, the non-adhesive band 710 covers the central portion of the user's mouth, the parafiltrum adhesive part 720 is adhered to an area between the nose and upper lip of the user, and the chin adhesive part 730 is adhered to a mid portion of the users' chin and reaches a bottom of the chin. The parafiltrum adhesive part 720 and the chin adhesive part 730 do not contact the user's lips.

The third embodiment could have a base (not shown) placed on a second surface of the adhesive strip with non-adhesive band 700 opposing to the non-adhesive band 710 to provide sheet strength to maintain the shape of the adhesive strip with non-adhesive band 700. The third embodiment also can use a releasing support as the releasing tab 230 as shown in Figs. 2A1 to Fig. 2D instead of the base. The constitution layers of the adhesive strip with non-adhesive band 700 can be replaced by those as shown in Figs. 5A to 5C.

Figs. 7B to 7E demonstrate front view of application diagrams of first to fourth variations of the third embodiment. Fig. 7B shows an unsymmetrical adhesive strip being part of the invention with non-adhesive band 701 which comprises of a parafiltrum adhesive part 721, a chin adhesive part 731, and a non-adhesive band 711 across a mid portion of a first surface of the adhesive strip with non-adhesive band 701. The parafiltrum adhesive part 721 may have a width comparable to the width of the lips to increase adhesion. The parafiltrum adhesive part 721 may further have a concave edge 741 to accommodate the shape of the nose. The parafiltrum adhesive part 721 may also have one or more cuts 751 which may improve its conformity to various contours of different user's parafiltrum. The non-adhesive band 711 has a height larger than a combined thickness of the user's lips in a closed condition. The non-adhesive band 711 is smaller than the width of the lips, and preferably about or smaller than 1/3 of the width of the lips. The chin adhesive part 731 may extend from the non-adhesive band 711 downwardly below the lips. The chin adhesive part 731 may have a width smaller than the width of the lips. The chin adhesive part 731 may have an offset L' from the vertical center line of the lips. The offset L' is preferably about 1/3 of the width of the lips to leave opening at the center of the mouth. The adhesive strip 700 may have unsymmetrical edges 761 and 771. The thickness and materials of the non-adhesive band 711 are the same with the non-adhesive band 710.

In usage, the non-adhesive band 711 covers a partial portion of the user's mouth, and the vertical center line of the non-adhesive band 711 may have an offset L' from the vertical center line of the user's lips, and the offset L' is satisfied with a relation of 0≦L'≦(1/2)W, wherein W is a width of the user's mouth. The parafiltrum adhesive part 721 is adhered to the area between the nose and the upper lip. The chin adhesive part 731 is adhered to the chin and reaches a bottom of the chin. The parafiltrum adhesive part 721 and chin adhesive part 731 do not contact with the user's lips.

The first variation could have a base (not shown) placed on a second surface of the adhesive strip with non-adhesive band 701 opposing to the non-adhesive band 711 to provide sheet strength to maintain the shape of the adhesive strip with non-adhesive band 701. The first variation also can use a releasing support as the releasing tab 230 as shown in Figs. 2A1 to Fig. 2D instead of the base. The constitution layers of the adhesive strip with non-adhesive band 701 can be replaced by those as shown in Figs. 5A to 5C.

Fig. 7C shows the second variation, which is a symmetrical adhesive strip with non-adhesive band 702, comprises of a parafiltrum adhesive part 722, a chin adhesive part 732, and a non-adhesive band 712 across a mid portion of a first surface of the adhesive strip with non-adhesive band 702. The parafiltrum adhesive part 722 may have a width comparable to the width of the lips to increase adhesion. The parafiltrum adhesive part 722 may further have a concave edge 742 to accommodate the shape of the nose. The parafiltrum adhesive part 722 may also have one or more cuts 752 which may improve its conformity to various contours of different user's parafiltrum. The non-adhesive band 712 is smaller than the width of the lips, and preferably about or smaller than 1/3 of the width of the lips. The chin adhesive part 732 may extend from the non-adhesive band 712 downwardly and reach bottom of the chin. The chin adhesive part 732 may have expanding width with diverging edges 762 and 772. The thickness and materials of the non-adhesive band 712 are the same with the non-adhesive band 710.

In usage, the non-adhesive band 712 covers a central portion of the user's mouth. The parafiltrum adhesive part 722 is adhered to the area between the nose and the upper lip. The chin adhesive part 732 is adhered to the chin and reaches a bottom of the chin. The parafiltrum adhesive part 721 and chin adhesive part 731 do not contact with the user's lips.

The second variation could have a base (not shown) placed on a second surface of the adhesive strip with non-adhesive band 702 opposing to the non-adhesive band 712 to provide sheet strength to maintain the shape of the adhesive strip with non-adhesive band 702. The second variation also can use a releasing support as the releasing tab 230 as shown in Figs. 2A1 to Fig. 2D instead of the base. The constitution layers of the adhesive strip with non-adhesive band 702 can be replaced by those as shown in Figs. 5A to 5C.

Fig. 7D shows the third variation, which is a symmetrical adhesive strip with non-adhesive band 703, comprises of a parafiltrum adhesive part 723, a chin adhesive part 733, and a non-adhesive band 713 across a mid portion of a first surface of the adhesive strip with non-adhesive band 703. The parafiltrum adhesive part 723 may have a width comparable to the width of the lips to increase adhesion. The parafiltrum adhesive part 723 may further have a concave edge 743 to accommodate the shape of the nose. The parafiltrum adhesive part 723 may also have one or more cuts 753 on its upper and/or bottom portion which may improve its conformity to various contours of different user's parafiltrum. The non-adhesive band 713 is smaller than the width of the lips, and preferably about or smaller than 1/3 of the width of the lips. A height of the non-adhesive band 713 is larger than a combined thickness of the user's lips in a closed condition. The chin adhesive part 733 may extend from the non-adhesive band 713 downwardly and reach bottom of the chin. The chin adhesive part 733 may have expanding width with convex edges 763 and 773. The chin adhesive part 733 may further have one or more cuts 783 which may improve its conformity to various contours of different user's chin. The thickness and materials of the non-adhesive band 713 are the same with those of the non-adhesive band 710 of the third embodiment.

In usage, the non-adhesive band 713 covers a central portion of the user's mouth. The parafiltrum adhesive part 722 is adhered to the area between the nose and the upper lip. The chin adhesive part 732 is adhered to a mid portion of the chin and reaches a bottom of the chin. The parafiltrum adhesive part 722 and chin adhesive part 732 do not contact with the user's lips.

The third variation could have a base (not shown) placed on a second surface of the adhesive strip with non-adhesive band 703 opposing to the non-adhesive band 713 to provide sheet strength to maintain the shape of the adhesive strip with non-adhesive band 703. The third variation also can use a releasing support as the releasing tab 230 as shown in Figs. 2A1 to Fig. 2D instead of the base. The constitution layers of the adhesive strip with non-adhesive band 703 can be replaced by those as shown in Figs. 5A to 5C.

Fig. 7E shows a fourth variation, which is a symmetrical adhesive strip 704 with non-adhesive band, comprises of a parafiltrum adhesive part 724, a chin adhesive part 734, and a non-adhesive band 714 across a mid portion of a first surface of the adhesive strip 704 with non-adhesive band. The parafiltrum adhesive part 724 may have a width comparable to a width of the lips to increase adhesion. The parafiltrum adhesive part 724 may further have a concave edge 744 to accommodate the shape of the nose. The parafiltrum adhesive part 724 may also have one or more cuts 754 on its upper and/or bottom portion which may improve its conformity to various contours of different user's parafiltrum. The non-adhesive band 714 is smaller than the width of the lips, and preferably about or smaller than 1/3 of the width of the lips. The chin adhesive part 734 may extend from the non-adhesive band 714 downwardly and reach bottom of the chin. The chin adhesive part 734 may have expanding width with convex edges 764 and 774. The chin adhesive part 734 may further have multiple cuts 784 or opening(s) 794 which may improve its conformity to various contours of different user's chin. The thickness and materials of the non-adhesive band 713 are the same with those of the non-adhesive band 710 of the third embodiment.

In usage, the non-adhesive band 714 covers a central portion of the user's mouth. The parafiltrum adhesive part 724 is adhered to the area between the nose and the upper lip. The chin adhesive part 734 is adhered to a mid portion of the chin and reaches a bottom of the chin. The parafiltrum adhesive part 724 and chin adhesive part 732 do not contact with the user's lips.

The fourth variation could have a base (not shown) placed on a second surface of the adhesive strip with non-adhesive band 704 opposing to the non-adhesive band 714 to provide sheet strength to maintain the shape of the adhesive strip with non-adhesive band 704. The fourth variation also can use a releasing support as the releasing tab 230 as shown in Figs. 2A1 to Fig. 2D instead of the base. The constitution layers of the adhesive strip with non-adhesive band 704 can be replaced by those as shown in Figs. 5A to 5C.

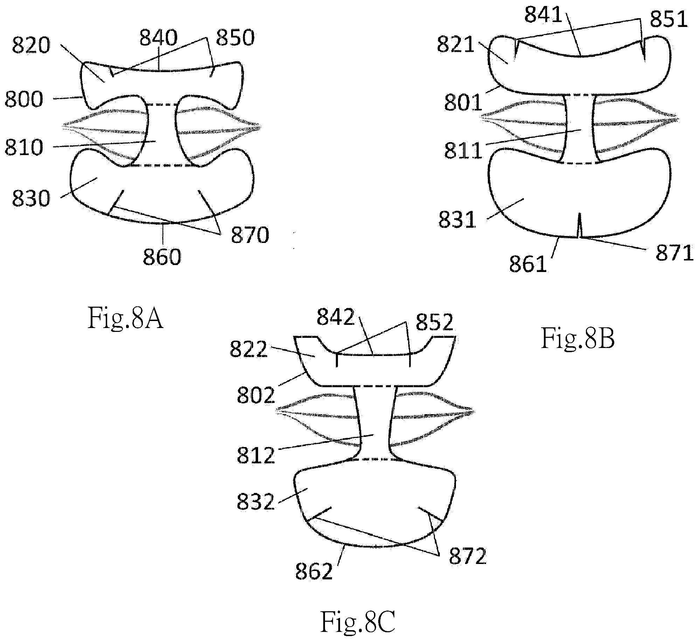

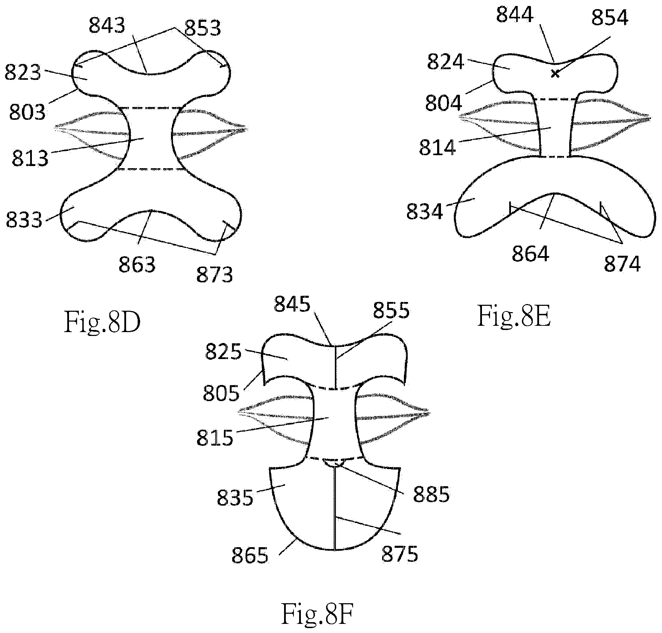

Fig. 8A demonstrates a front view of an application diagram of an adhesive strip with non-adhesive band 800 according to a fourth embodiment of the present invention. Figs. 8B to 8F show front views of application diagrams of first to fifth variations of the fourth embodiment. Figs. 8A to 8F respectively show an adhesive strip (800, 801, 802, 803, 804, 805) comprises of a parafiltrum adhesive part (820, 821, 822, 823, 824, 825), a chin adhesive part (830, 831, 832, 833, 834, 835), and a non-adhesive band (810, 811, 812, 813, 814, 815) across a central portion of a first surface of the adhesive strip (800, 801, 802, 803, 804, 805). The parafiltrum adhesive parts (820, 821, 822, 823, 824, 825) may have a width comparable to the width of the lips to increase adhesion. The parafiltrum adhesive part (820, 821, 822, 823, 824, 825) may further have a concave edge (840, 841, 842, 843, 844, 845) to accommodate the shape of the nose. The parafiltrum adhesive part (820, 821, 822, 823, 824, 825) may also have one or more cuts (850, 851, 852, 853, 854, 855) which may improve its conformity to various contours of the parafiltrum. The non-adhesive band (810, 811, 812, 813, 814, 815) is smaller than the width of the lips, and preferably about or smaller than 1/3 of the width of the lips. The non-adhesive band (810, 811, 812, 813, 814, 815) may have a height larger than a combined thickness of the lips in a closed condition. The chin adhesive part (830, 831, 832, 833, 834, 835) may have a width comparable to the width of the lips. The chin adhesive part (830, 831, 832, 835) may extend from the non-adhesive band (810, 811, 812, 815) downwardly with convex bottom edges (860, 861, 862, 865). The chin adhesive part (833, 834) may extend from the non-adhesive band (813, 814) downwardly with convex bottom edges (863, 864). The chin adhesive part (830, 831, 832, 833, 834, 835) may further have multiple cuts (870, 871, 872, 873, 874, 875) or opening(s) (885) which may improve its conformity to various contours of different user's chin. Besides, the fourth embodiment and the first to fifth variations could be unsymmetrical adhesive strip with non-adhesive band. The non-adhesive band (810, 811, 812, 813, 814, 815) may further have an offset (not shown) from the vertical center line of the lips to leave opening at the center of the mouth. That is, in usage, the non-adhesive band (810, 811, 812, 813, 814, 815) may have an offset L from the central portion of the lips so that an unblocked flow passage between the upper and lower lips is formed when the user intends to open the mouth to breathe, cough or speak. The non-adhesive band (810, 811, 812, 813, 814, 815) can be made of elastic materials with various thickness and flexibility. Potential materials of the non-adhesive band (810, 811, 812, 813, 814, 815) include polyurethane (PU), polyethylene (PE), silicone, cloth, non-woven cloth, polyethylene terephthalate (PET), paper, as well as other flexible thin films. In an optional implementation, the surface of the non-adhesive band (810, 811, 812, 813, 814, 815) contacting with the user's lips may be a low-friction surface with a friction coefficient less than 0.5 to allow the lips to move more freely when the user intends to open the mouth to breathe, cough or speak. Moisture may cause reduction of adhesiveness of the adhesive strip (800, 801, 802, 803, 804, 805) and, in turn, cause the adhesive strip (800, 801, 802, 803, 804, 805) losing its capability to close the mouth without air leakage. The surface of the non-adhesive band (810, 811, 812, 813, 814, 815) contacting the user's lips may be a hydrophobic surface that repels liquid (like saliva) and prevents it from entering the interface between the adhesive strip (800, 801, 802, 803, 804, 805) and the skin, and preventing the reduction of adhesiveness of the adhesive strip (800, 801, 802, 803, 804, 805).

In usage, the non-adhesive band (810, 811, 812, 813, 814, 815) covers a central portion of the user's mouth. The parafiltrum adhesive part (820, 822, 823, 824, 825) is adhered to the area between the nose and the upper lip. The chin adhesive part (830, 831, 832, 833, 834, 835) is adhered to the chin. The parafiltrum adhesive part (820, 822, 823, 824, 825) and chin adhesive part (830, 831, 832, 833, 834, 835) do not contact with the user's lips.

The fourth embodiment and its first to fifth variations could have a base (not shown) placed on a second surface of the adhesive strip (800, 801, 802, 803, 804, 805) opposing to the non-adhesive band (810, 811, 812, 813, 814, 815) to provide sheet strength to maintain the shape of the adhesive strip (800, 801, 802, 803, 804, 805). The fourth embodiment and its first to fifth variations also can use a releasing support as the releasing tab 230 as shown in Figs. 2A1 to Fig. 2D instead of the base. The constitution layers of the adhesive strip with non-adhesive band 704 can be replaced by those as shown in Figs. 5A to 5C.

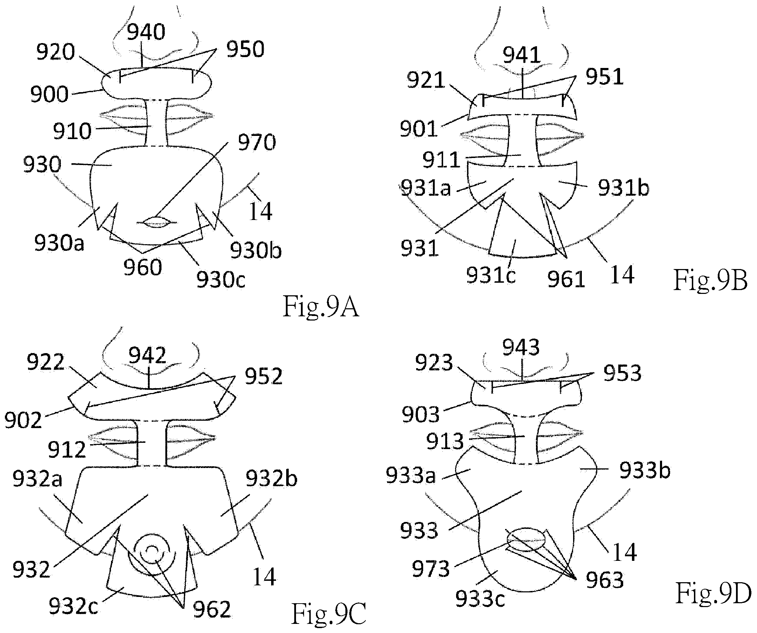

Fig. 9A demonstrates a front view of an application diagram of an adhesive strip with non-adhesive band 900 according to a fifth embodiment of the present invention. Figs. 9B to 9D show front views of application diagrams of first to third variations of the fifth embodiment. Figs. 9A to 9D respectively show an adhesive strip with non-adhesive band (900, 901, 902, 903) comprises of a parafiltrum adhesive part (920, 921, 922, 923), a chin adhesive part (930, 931, 932, 933), and a non-adhesive band (910, 911, 912, 913) across a central portion of a first surface of the adhesive strip (900, 901, 902, 903). The parafiltrum adhesive parts (920, 921, 922, 923) may have a width comparable to the width of the lips to increase adhesion. The parafiltrum adhesive part (920, 921, 922, 923) may further have a convex edge (940), concave edge (941, 942), or a flat edge (943) to accommodate the shape of the nose. The parafiltrum adhesive part (920, 921, 922, 923) may also have one or more cuts (950, 951, 952, 953) which may improve its conformity to various contours of the parafiltrum. The non-adhesive band (910, 911, 912, 913) is smaller than the width of the lips, and preferably about or smaller than 1/3 of the width of the lips. A height of the non-adhesive band (910, 911, 912, 913) is larger than a combined thickness of the lips in a closed condition. The chin adhesive part (930, 931, 932, 933) may have a width comparable to the width of the lips with a left extension (930a, 931a, 932a, 933a) and a right extension (930b, 931b, 932b, 933b). The chin adhesive part (930, 931, 932, 933) may extend from the non-adhesive band (910, 911, 912, 913) downwardly and reach bottom of the chin with a bottom extension (930c, 931c, 932c, 933c). The chin adhesive part (930, 931, 932, or 933) may further have multiple cuts (960, 961, 962, or 963) or opening(s) (973) which may improve its conformity to various contours of different user's chin. Besides, the fifth embodiment and its first to third variations could be unsymmetrical adhesive strips with non-adhesive bands. The non-adhesive band (910, 911, 912, 913) could have an offset (not shown) from the vertical center line of the adhesive strip (900, 901, 902, 903). That is, in usage, the non-adhesive band (910, 911, 912, 913) may further have an offset L from the vertical center line of the lips so that an unblocked flow passage between the upper and lower lips is formed when the user intends to open the mouth to breathe, cough or speak. The non-adhesive band (910, 911, 912, 913) can be made of elastic materials with various thickness and flexibility. Potential materials of the non-adhesive band (910, 911, 912, 913) include polyurethane (PU), polyethylene (PE), silicone, cloth, non-woven cloth, polyethylene terephthalate (PET), paper, as well as other flexible thin films. In an optional implementation, the surface of the non-adhesive band (910, 911, 912, 913) contacting with the user's lips may be a low-friction surface with a friction coefficient less than 0.5 to allow the lips to move more freely when the user intends to open the mouth to breathe, cough or speak. Moisture may cause reduction of adhesiveness of the adhesive strip (900, 901, 902, 903) and, in turn, cause the adhesive strip (900, 901, 902, 903) losing its capability to close the mouth without air leakage. The surface of the non-adhesive band (910, 911, 912, 913) contacting the user's lips may be a hydrophobic surface that repels liquid (like saliva) and prevents it from entering the interface between the adhesive strip (900, 901, 902, 903) and the skin, and preventing the reduction of adhesiveness of the adhesive strip (900, 901, 902, 903).

In usage, the non-adhesive band (910, 911, 912, 913) covers a central portion of the user's mouth. The parafiltrum adhesive part (920, 921, 922, 923) is adhered to the area between the nose and the upper lip. The chin adhesive part (930, 931, 932, 933) is adhered to the chin. The parafiltrum adhesive part (920, 921, 922, 923) and chin adhesive part (930, 931, 932, 933) do not contact with the user's lips.

The fifth embodiment and its first to third variations could have a base (not shown) placed on a second surface of the adhesive strip (900, 901, 902, 903) opposing to the non-adhesive band (910, 911, 912, 913) to provide sheet strength to maintain the shape of the adhesive strip (900, 901, 902, 903). The fifth embodiment and its first to third variations also can use a releasing support as the releasing tab 230 as shown in Figs. 2A1 to Fig. 2D instead of the base. The constitution layers of the adhesive strip with non-adhesive band (900, 901, 902, 903) can be replaced by those as shown in Figs. 5A to 5C.

The devices of the present invention may further be used in combination with other apparatus for the treatment of sleep-disordered breathing. For example, the adhesive strips with non-adhesive band described herein may be used in combination with a negative oral pressure therapy device (NOPT), such as those described in, for example, U.S. Patent Ser. No.7,918,222 B2, filed Mar. 28, 2007, for applying negative pressure to the oral cavity. In this way, with the device of the present invention to prevent mouth from opening during sleep, and the NOPT device increases negative pressure in the oral cavity, the pressure gradient between the airway and the oral cavity can be maintained without leakage from the mouth opening, thus urging the soft palate toward the oral cavity and the tongue toward the upper hard palate to clear upper airway. In addition the adhesive strips with non-adhesive band of the present invention may be used in combination with one-way nasal valve devices, such as those described in, for example, U.S. Patent Ser. No. 8,061,357, filed Jun. 18, 2008, and Ser. No. 8,020,700, filed Feb. 24, 2010. By increasing airway pressure using such nasal valve devices while preventing mouth leakage in the oral cavity using the present invention, the pressure gradient from airway to the oral cavity is maintained, thus urging the soft palate toward the oral cavity and the tongue toward the upper hard palate to clear upper airway. The adhesive strips with non-adhesive band of the present invention may further be used in combination with a conventional constant positive airway pressure (CPAP) apparatus for delivering air under positive pressure to the nasal airway. With the device of the present invention keeping mouth closing and preventing air leakage in the oral cavity, and the CPAP apparatus increasing Pressure in the airway, the pressure gradient between the airway and the oral cavity is maintained, thus urging soft palate toward the oral cavity and the tongue toward the upper hard palate to clear upper airway.

In addition to the specific uses described above, other embodiments and uses of the invention will be apparent to those skilled in the art from consideration of the specification and practice of the invention disclosed herein. The specification and examples should be considered exemplary only with the true scope of the invention indicated by the following claims. As will be easily understood by those of ordinary skill in the art, variations and modifications of each of the disclosed embodiments can be easily made within the scope of this invention as defined by the following claims.

## Claims

1. An adhesive strip with non-adhesive band, the adhesive strip with non-adhesive band comprising:
a strip body (120, 200, 401, 402, 403, 404) having a first surface being adhesive and a second surface; and
a non-adhesive band (110, 210, 410, 420, 430, 440) disposed on the first surface of the strip body to divide the strip body (120, 200, 401, 402, 403, 404) into an upper adhesive part and a lower adhesive part, wherein the non-adhesive band has an outer contour conforms to a contour of the strip body within their overlapping configuration and a height of the non-adhesive band is larger than a combined thickness of an upper and lower lips of a user in a mouth-closed condition when applied to the user's mouth, the non-adhesive band has a width W smaller than a width of the user's mouth,
**characterized in that**
the non-adhesive band (110, 210, 410, 420, 430, 440) is configured to offset from a central portion of the user's mouth to provide an obstructive mouth opening area (110a) covered by the non-adhesive band (110, 210, 410, 420, 430, 440) besides a non-obstructive mouth opening area (110b) when applied to the user's mouth; and
the configuration of the strip body is selected from one of the following: the strip body (120, 200) has a concave edge (170) and a convex edge (180) opposing thereto and forming a protruding tab (190) on the non-adhesive band (110, 210), the strip body (401) has two unsymmetrical edges (4011, 4012) with one of the two edges having a protruding tab (4101) on the non-adhesive band (410), the strip body (402) has two symmetrical edges (4021, 4022) forming a pair of opposing protrusions on the non-adhesive band (420), the strip body (403) has two opposing zigzag edges (4031, 4032), and the strip body (404) has a concave edge and a convex edge opposing thereto and forming a protruding tab on the non-adhesive band (440) and a non-adhesive tab (4043) at one of top and bottom edges of the strip body.

2. An adhesive strip with non-adhesive band, the adhesive strip with non-adhesive band comprising:
a strip body (701) having a first surface being adhesive and a second surface; and
a non-adhesive band (711) disposed on the first surface of the strip body to divide the strip body into an upper adhesive part (721) and a lower adhesive part (761), wherein the non-adhesive band has an outer contour conforms to a contour of the strip body within their overlapping configuration and a height of the non-adhesive band is larger than a combined thickness of an upper and lower lips of a user in a mouth-closed condition when applied to the user's mouth, the non-adhesive band has a width W smaller than a width of the user's mouth,
**characterized in that**
the non-adhesive band (711) is configured to offset from a central portion of the user's mouth to provide an obstructive mouth opening area (110a) covered by the non-adhesive band (711) besides a non-obstructive mouth opening area (110b) when applied to the user's mouth;
the strip body (701) has an unsymmetrical configuration and the upper adhesive part is a parafiltrum adhesive part (721) with a width similar to a width of the user's lips and the lower adhesive part is a chin adhesive part (731), and the non-adhesive band (711) and the chin adhesive part (731) are formed near an edge of the strip body (701).

3. The adhesive strip with non-adhesive band of claim 1 or 2, which is **characterized in that** the strip body is constituted from one of the following: an adhesive strip (120) connecting to the non-adhesive band (110) and a base (140) underlying the adhesive strip, an adhesive strip (520) and a base (500a) underlying the adhesive strip and an adhesive band (510') having an outer contour conforming to the contour of the non-adhesive band (510) and placed between the adhesive strip and the non-adhesive band, and a non-adhesive base (504a) and an upper adhesive part (524a) and a lower adhesive part (524b) over the non-adhesive base and a partial surface of the non-adhesive base being exposed between the upper adhesive part and the lower adhesive part.

4. The adhesive strip with non-adhesive band of claim 1 or 2, which is **characterized in that** the upper and lower adhesive parts have thinner edges (623a, 633a) than their other portions.

5. The adhesive strip with non-adhesive band of claim 1 or 2, which is **characterized in that** the strip body (200) comprises an adhesive strip connecting to the non-adhesive band (210) and a releasing support (230) under the adhesive strip facing the non-adhesive band (210).

6. The adhesive strip with non-adhesive band of claim 5, which is **characterized in that** the releasing support (230) comprises an upper releasing support and a lower releasing support symmetrically disposed to each other, a notch (234) and an applying tab (232) are formed at an interface of the upper and lower releasing supports and align to a horizontal center line of the non-adhesive band (210).

7. The adhesive strip with non-adhesive band of claim 2, which is **characterized in that** the lower adhesive part (731) is longer than the upper adhesive part (721).

8. The adhesive strip with non-adhesive band of claim 1 or 2, which is **characterized in that** the non-adhesive band (110, 210, 410, 420, 430, 440, 711) is made of elastic materials.

9. The adhesive strip with non-adhesive band of claim 1 or 2, which is **characterized in that** the non-adhesive band (110, 210, 410, 420, 430, 440, 711) has a low-friction surface with a friction coefficient less than 0.5, or a hydrophobic surface.

## Patentansprüche

1. Klebestreifen mit nicht-klebendem Band, der Klebestreifen mit nicht-klebendem Band umfasst:
Einen Streifenkörpers (120, 200, 401, 402, 403, 404) mit einer ersten klebenden Oberfläche und einer zweiten Oberfläche; und
ein auf einer ersten Oberfläche des Streifenkörpers angeordnetes nichtklebendes Band (110, 210, 410, 420, 430, 440), um den Streifenkörper (120, 200, 401, 402, 403, 404) in einen oberen klebenden Teil und einen unteren klebenden Teil aufzuteilen, wobei das nicht-klebende Band einer äußeren Kontur des Streifenkörpers innerhalb ihrer überlappenden Ausgestaltung entspricht und eine Höhe des nicht-klebenden Bands größer ist als die kombinierte Dicke der unteren und oberen Lippen eines Benutzers bei geschlossenem Mund, wenn auf den Mund des Benutzers angewandt, das nicht-klebende Band hat eine Breite W kleiner als eine Breite des Munds des Benutzers,
**dadurch gekennzeichnet, dass**
das nicht-klebende Band (110, 210, 410, 420, 430, 440) ausgebildet ist, um zu einem zentralen Abschnitt des Munds des Benutzers versetzt zu sein und einen obstruktiven Mundöffnungsbereich (110a) bereitzustellen, welcher durch das nicht-klebende Band (110, 210, 410, 420, 430, 440) mit Ausnahme eines nicht obstruktiven Mundöffnungsbereichs (110b) bedeckt ist, wenn auf den Mund des Benutzers angewendet; und
die Ausbildung des Streifenkörpers wird aus einer der folgenden ausgewählt: der Streifenköper (120, 200) hat eine konkave Kante (170) und eine dieser gegenüberliegende und eine hervorstehende Lasche (190) an dem nicht-klebenden Band (110, 210) ausbildende konvexe Kante (180), der Streifenkörper (401) hat zwei unsymmetrische Kanten (4011, 4012), wobei eine der zwei Kanten eine herausstehende Lasche (4101) an dem nicht-klebendem Band (410) aufweist, der Streifenkörper (402) hat zwei symmetrische Kanten (4021, 4022), die ein Paar von gegenüberliegenden Vorsprüngen an dem nicht-klebendem Band (420) ausbilden, der Streifenkörper (403) hat zwei gegenüberliegende Zickzack-Kanten (4031, 4032), und der Streifenkörper (404) hat eine konkave Kante und eine dieser gegenüberliegende konvexe Kante, welche eine hervorstehende Lasche an dem nicht-klebenden Band (440) und eine nicht-klebende Lasche (4043) an einer der oberen oder unteren Kanten des Streifenkörper ausbildet.

2. Klebestreifen mit nicht-klebendem Band, der Klebestreifen mit nicht-klebendem Band umfasst:
Einen Streifenkörper (701) mit einer ersten klebenden Oberfläche und einer zweiten Oberfläche; und
ein auf einer ersten Oberfläche des Streifenkörpers angeordnetes nichtklebendes Band (711), um den Streifenkörper in einen oberen klebenden Teil (721) und einen unteren klebenden Teil (761) aufzuteilen, wobei das nicht-klebende Band einer äußeren Kontur des Streifenkörpers innerhalb ihrer überlappenden Ausgestaltung entspricht und eine Höhe des nicht-klebenden Bands größer ist als die kombinierte Dicke der unteren und oberen Lippen eines Benutzers bei geschlossenem Mund, wenn auf den Mund des Benutzers angewandt, das nicht-klebende Band hat eine Breite W kleiner als eine Breite des Munds des Benutzers,
**dadurch gekennzeichnet, dass**
das nicht-klebende Band (711) ausgebildet ist, um zu einem zentralen Abschnitt des Munds des Benutzers versetzt zu sein und einen obstruktiven Mundöffnungsbereich (110a) bereitzustellen, welcher durch das nicht-klebende Band (711) mit Ausnahme eines nicht obstruktiven Mundöffnungsbereichs (110b) bedeckt ist, wenn auf den Mund des Benutzers angewendet; und
der Streifenkörper (701) hat eine unsymmetrische Ausbildung und der obere klebende Teil ist ein Parafiltrum-Klebeteil (721) mit einer Breite entsprechend einer Breite der Lippen des Benutzers und der untere Klebeteil ist ein Kinnklebeteil (731), und das nicht-klebende Band (711) und der Kinnklebeteil (731) sind nahe der Kanten des Streifenkörper (701) gebildet.

3. Klebestreifen mit nicht-klebendem Band nach Anspruch 1 oder 2, **dadurch gekennzeichnet, dass** der Streifenkörper gebildet ist aus einem der folgenden: Ein Klebestreifen (120), der mit dem nicht-klebenden Band (110) verbunden ist und einer dem Klebestreifen unterliegenden Basis (140), ein Klebestreifen (520) und eine dem Klebestreifen unterliegende Basis (500a) und ein zwischen dem Klebestreifen und dem nicht-klebenden Band angeordnetes Klebeband (510') mit einer Außenkontur entsprechend der Kontur des nicht-klebenden Bands (510), und eine nicht-klebende Basis (504a) und ein oberes Klebeteil (524a) und ein unteres Klebeteil (524b) über der nicht-klebenden Basis, wobei eine Teilfläche der nicht-klebenden Basis zwischen dem oberen Klebeteil und dem unteren Klebeteil freiliegt.

4. Klebestreifen mit nicht-klebendem Band nach Anspruch 1 oder 2, **dadurch gekennzeichnet, dass** der obere und untere Klebeteil dünnere Kanten (623a, 633a) als ihre anderen Abschnitte haben.

5. Klebestreifen mit nicht-klebendem Band nach Anspruch1 oder 2, **dadurch gekennzeichnet, dass** der Streifenkörper (200) einen mit dem nicht-klebenden Band (210) verbindenden Klebestreifen und eine unter dem Klebestreifen angeordnete und dem nicht-klebenden Band (210) zugewandten Trennauflage aufweist.

6. Klebestreifen mit nicht-klebendem Band nach Anspruch 5, **dadurch gekennzeichnet, dass** die Trennauflage (230) eine obere Trennauflage und eine untere Trennauflage aufweist, welche symmetrisch zueinander angeordnet sind, wobei an einer Schnittstelle zwischen oberer und unterer Trennauflage ein Einschnitt (234) und eine Anwendungslasche (232) ausgebildet sind und auf eine horizontalen Zentrallinie des nicht-klebenden Bands (210) ausgerichtet sind.

7. Klebestreifen mit nicht-klebendem Band nach Anspruch 2, **dadurch gekennzeichnet, dass** das untere Klebeteil (731) länger ist als das obere Klebeteil (721).

8. Klebestreifen mit nicht-klebendem Band nach Anspruch 1 oder 2, **dadurch gekennzeichnet, dass** das nicht-klebende Band (110, 210, 410, 420, 430, 440, 711) aus einem elastischen Material gemacht ist.

9. Klebestreifen mit nicht-klebendem Band nach Anspruch 1 oder 2, **dadurch gekennzeichnet, dass** das nicht-klebende Band (110, 210, 410, 420, 430, 440, 711) eine friktionsarme Oberfläche mit einem Friktionskoeffizienten kleiner als 0,5 oder eine hydrophobe Oberfläche hat.

## Revendications

1. Ruban adhésif comportant une bande non adhésive, le ruban adhésif avec la bande non adhésive comprenant :
un corps de ruban (120, 200, 401, 402, 403, 404) présentant une première surface qui est adhésive et une seconde surface ; et
une bande non adhésive (110, 210, 410, 420, 430, 440), disposée sur la première surface du corps de ruban de manière à diviser le corps de ruban (120, 200, 401, 402, 403, 404) en une partie adhésive supérieure et une partie adhésive inférieure, dans lequel la bande non adhésive présente un contour externe qui se conforme à un contour du corps de ruban dans leur configuration en recouvrement et une hauteur de la bande non adhésive est supérieure à une épaisseur combinée des lèvres supérieure et inférieure d'un utilisateur ayant la bouche fermée lorsqu'elle est appliquée sur la bouche de l'utilisateur, la bande non adhésive présentant une largeur W inférieure à une largeur de la bouche de l'utilisateur,
**caractérisé en ce que**
la bande non adhésive (110, 210, 410, 420, 430, 440) est configurée de manière décalée par rapport à une partie centrale de la bouche de l'utilisateur afin de former une zone d'ouverture de bouche obstructive (110a) recouverte par la bande non adhésive (110, 210, 410, 420, 430, 440) à côté d'une zone d'ouverture de bouche non obstructive (110b) lorsqu'elle est appliquée sur la bouche de l'utilisateur ; et
la configuration du corps de ruban est sélectionnée parmi l'une des suivantes : le corps de ruban (120, 200) présente un bord concave (170) et un bord convexe (180) opposé à ce dernier et formant une patte en saillie (190) sur la bande non adhésive (110, 210), le corps de ruban (401) présente deux bords non symétriques (4011, 4012), l'un des deux bords présentant une patte en saillie (4101) sur la bande non adhésive (410), le corps de ruban (402) comporte deux bords symétriques (4021, 4022) formant une paire de saillies opposées sur la bande non adhésive (420), le corps de ruban (403) présente deux bords en zigzag opposés (4031, 4032), et le corps de ruban (404) comporte un bord concave et un bord convexe opposé à ce dernier et formant une patte en saillie sur la bande non adhésive (440) et une patte non adhésive (4043) au niveau de l'un des bords supérieur et inférieur du corps de ruban.

2. Ruban adhésif comportant une bande non adhésive, le ruban adhésif avec la bande non adhésive comprenant :
un corps de ruban (701) présentant une première surface qui est adhésive et une seconde surface ; et
une bande non adhésive (711) disposée sur la première surface du corps de ruban de manière à diviser le corps de ruban en une partie adhésive supérieure (721) et une partie adhésive inférieure (761), dans lequel la bande non adhésive présente un contour externe conforme à un contour du corps de ruban dans leur configuration en recouvrement et une hauteur de la bande non adhésive est supérieure à une épaisseur combinée des lèvres supérieure et inférieure d'un utilisateur ayant la bouche fermée lorsqu'elle est appliquée sur la bouche de l'utilisateur, la bande non adhésive présente une largeur W inférieure à une largeur de la bouche de l'utilisateur,
**caractérisé en ce que**
la bande non adhésive (711) est configurée de manière à être décalée par rapport à une partie centrale de la bouche de l'utilisateur afin de former une zone d'ouverture de bouche obstructive (110a) recouverte par la bande non adhésive (711) à côté d'une zone d'ouverture de bouche non obstructive (110b) lorsqu'elle est appliquée sur la bouche de l'utilisateur ;
le corps de ruban (701) présente une configuration non symétrique et la partie adhésive supérieure est une partie adhésive pour le philtrum (721) avec une largeur similaire à une largeur des lèvres de l'utilisateur et la partie adhésive inférieure est une partie adhésive pour le menton (731), et la bande non adhésive (711) et la partie adhésive pour le menton (731) sont formées à proximité d'un bord du corps de ruban (701).

3. Ruban adhésif comportant une bande non adhésive selon la revendication 1 ou 2, caractérisé en que le corps de ruban est constitué de l'un des suivants : un ruban adhésif (120) reliant la bande non adhésive (110) et une base (140) au-dessous du ruban adhésif, un ruban adhésif (520) et une base (500a) au-dessous du ruban adhésif et un ruban adhésif (510') présentant un contour externe se conformant au contour de la bande non adhésive (510) et placé entre le ruban adhésif et la bande non adhésive, et une base non adhésive (504a) et une partie adhésive supérieure (524a) et une partie adhésive inférieure (524b) au-dessus de la base non adhésive et une partie de la surface de la base non adhésive étant exposée entre la partie adhésive supérieure et la partie adhésive inférieure.

4. Ruban adhésif comportant une bande non adhésive selon la revendication 1 ou 2, qui est **caractérisé en ce que** les parties adhésives supérieure et inférieure présentent des bords plus minces (623a, 633a) que leurs autres parties.

5. Ruban adhésif comportant une bande non adhésive selon la revendication 1 ou 2, qui est **caractérisé en ce que** le corps de ruban (200) comprend un ruban adhésif reliant la bande non adhésive (210) et un support à séparer (230) au-dessous du ruban adhésif faisant face à la bande non adhésive (210).

6. Ruban adhésif comportant une bande non adhésive selon la revendication 5, qui est **caractérisé en ce que** le support à séparer (230) comprend un support à séparer supérieur et un support à séparer inférieur disposés de manière symétrique l'un par rapport à l'autre, une encoche (234) et une patte d'application (232) étant formées au niveau d'une interface des supports à séparer supérieur et inférieur et alignées suivant une ligne centrale horizontale de la bande non adhésive (210).

7. Ruban adhésif comportant une bande non adhésive selon la revendication 2, qui est **caractérisé en ce que** la partie adhésive inférieure (731) est plus longue que la partie adhésive supérieure (721).

8. Ruban adhésif comportant une bande non adhésive selon la revendication 1 ou 2, qui est **caractérisé en ce que** la bande non adhésive (110, 210, 410, 420, 430, 440, 711) est réalisée à base de matériaux élastiques.

9. Ruban adhésif comportant une bande non adhésive selon la revendication 1 ou 2, qui est **caractérisé en ce que** la bande non adhésive (110, 210, 410, 420, 430, 440, 711) présente une surface à faible coefficient de frottement avec un coefficient de frottement inférieur à 0,5, ou une surface hydrophobe.
